# DEMANDE DE BREVET EUROPEEN

(11) **EP 1 466 884 A1**
(43) Date de publication de la demande: **13.10.2004**
(21) Numéro de dépôt: 03290865.9
(22) Date de dépôt: 08.04.2003
(51) Int. Cl.: C07C 39/21, C07C 37/055, C07C 37/20

(54) **Procédé de préparation du resvératrol et ses applications**

(71) Demandeur: Sochinaz SA, 1895 Vionnaz (CH)
(72) Inventeur: Haider, Akhtar, 1024 Eculens (CH); Megavand, Sophie, 1024 Ecublens (CH); Fadi, Fabrice, 1926 Fully (CH)
(74) Mandataire: Burtin, Jean-François

(57) **Abrégé**

La présente invention se situe dans le domaine de la chimie et plus particulièrement dans le domaine de la chimie organique de synthèse.

L'invention à pour objet un nouveau procédé du Resvératrol ou 3,5-dihydroxy-4'-hydroxystilbène (isomère E) qui consiste en ce qu'on effectue la déprotection des groupes phénol au départ du 3,5-dibenzyloxy 4'-benzyloxy stilbène, au moyen du couple halogénure d'aluminium / N, N-dialkylaniline, pour obtenir le 3,5-dihydroxy 4'-hydroxy stilbène avec un degré de pureté supérieurs à 98%.

Le 3,5-dibenzyloxy 4'-benzyloxystilbène (isomère E) est lui même préparé par réaction du Wittg-Horner entre le 4-benzyloxyphényl phosphonate de diéthyle et le 3,5-dibenzyloxybenzaldéhyde.

Application au domaine de la cosmétique et de la thérapeutique.

## Description

La présente invention se rapporte au domaine de la chimie organique et plus particulièrement au domaine de la chimie thérapeutique.

Elle a plus particulièrement pour objet un nouveau procédé de synthèse d'un dérivé stilbénique polyhydroxylé.

Elle concerne plus précisément un nouveau procédé de production du 3,4',5-trihydroxy-trans-Stilbène (ou E)-dénommé communément Resvératrol, répondant à la formule 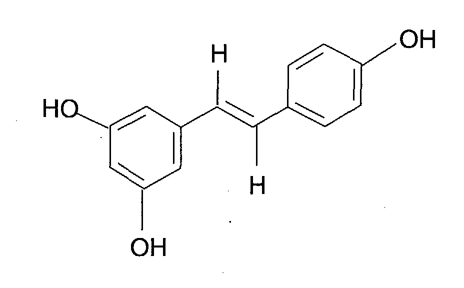 caractérisé en ce qu'on soumet un halogénure de p-hydroxybenzyle dont la fonction phénol est protégée, à l'action d'un phosphite de trialkyle en présence d'un iodure de métal alcalin puis met à réagir le sel de phosphonate d'alkyle ainsi obtenu selon une réaction de Wittig-Horner, avec un dihydroxybenzaldehyde dont les fonctions phénol sont protégées, en présence d'un agent basique pour former un trihydroxy stilbène bloqué dont on débloque les fonctions phénol sans endommager la double liaison carbone-carbone.

Il est certain que dans les présentes synthèses du Resvératrol, les auteurs se sont heurtés au problème du blocage puis du déblocage des fonctions phénol. Sans blocage des fonctions phénol, la réaction avec le phosphonate selon la réaction de Wittig-Horner donne de mauvais résultats sur le plan de la pureté du produit final et sur le plan de la spécificité. Il est donc important pour l'économie de ce procédé de préparation que les matières premières soient abondantes et bon marché, et facilement réactives et que les réactions ne soient pas susceptibles de conduire à des proportions importantes de sous-produits.

Les références antérieures comme le brevet PCT/US01/05167 au nom de Brigham Young University décrit un procédé de synthèse du Resvératrol en utilisant l'acide résorcylique comme produit de départ. Après blocage des fonctions phénol il faut réduire le carboxyle en fonction alcool à l'aide d'un hydrure complexe. Cette réaction est difficile et nécessite un temps de réaction prolongé avec formation de sous produits.

Le choix des réactifs de blocage n'est pas très large car l'utilisation de réactif de méthylation ou d'éthylation nécessite en fin de synthèse des réactions de déblocage avec l'iodure de phosphore ou l'acide iodhydrique ou l'acide bromhydrique qui peuvent affecter la double liaison du groupe stilbène et conduire à des réactions de clivage.

Au contraire, le blocage par un réactif allylique ou benzylique nécessite une réaction d'hydrogénolyse en fin de synthèse. Cette réaction d'hydrogénolyse s'effectue au moyen de l'hydrogène et d'un catalyseur à base de métal noble comme le platine, le palladium ou le rhodium. Il est très difficile d'effectuer cette hydrogénolyse sans toucher à la double liaison du stilbène et de ce fait le rendement en Resvératrol est faible car il se forme principalement un produit d'hydrogénation de la double liaison.

Une autre méthode de synthèse décrite également dans le brevet PCT/US01/05167 consiste à faire réagir un halogènure d'acide résorcylique avec l'acétoxystyrène. Ce type de réaction ne fournit pas de rendement satisfaisant.

Il restait donc à trouver une méthode de synthèse qui porte remède aux nombreux inconvénients constatés avec les procédés de l'art antérieur et qui conduise avec un bon rendement à un dérivé du stilbène ayant la configuration trans (ou E) de la chaîne stilbénique.

Ces problèmes techniques ont été résolus selon le procédé de la présente invention en partant d'un alcool *p*-hydroxybenzylique protégé, que l'on transforme en un halogénure puis en phosphonate (réaction d'Arbusov) et que l'on fait réagir dans les conditions de la réaction de Wittig-Horner avec un aldéhyde dihydroxylé protégé ' pour former le dérivé stilbénique ayant la configuration trans avec une stérospécificité très élevée.

La réaction de l'halogénure de benzyle avec un phosphite de trialkyle s'effectue sans solvant. La condensation du phosphonate avec un benzaldéhyde substitué s'effectue dans le THF ou le toluène en présence d'une base, donne naissance au trans (E) stilbène avec un rendement élevé.

Ce type de réaction en 2 étapes peut s'effectuer dans un seul réacteur (one pot reaction) en faisant réagir dans un premier temps le chlorure de benzyle et le phosphite d'alkyle puis en ajoutant le solvant, la base avec formation "in-situ" de l'ylure et enfin l'addition d'aldéhyde pour former le polyhydroxy stilbène. Il est également possible d'effectuer cette opération en deux étapes en formant d'abord l'ylure par réaction du phosphonate de *p*-hydroxyphényle avec un réactif fortement alcalin dans un solvant polaire, à isoler l'ylure puis à le faire réagir avec l'aldehyde hydroxylé pour obtenir le rendement le plus élevé possible en polyhydroxy stilbène. L'ylure est plus réactif et se condense avec un benzaldéhyde d'une manière pratiquement quantitative.

En général, le déblocage des fonctions phénol bloquées par un benzyle, s'effectue par hydrogénolyse.

Au contraire des réactions similaires décrites dans l'art antérieur, les Demandeurs se sont efforcé d'étudier les catalyseurs qui ne conduisent pas à une hydrogénation totale mais qui soient suffisamment actifs pour amener une déprotection complète des fonctions phénol.

C'est ainsi que l'hydrogénolyse en présence de charbon palladié conduit à une hydrogénation de la double liaison. L'hydrogénolyse en présence d'oxyde de palladium amène également la réduction de la double liaison. Les réactions d'échange avec le cyclohexène en présence d'hydroxyde de palladium sur charbon entraînent quelque soit le solvant, une réduction complète de la double liaison.

Les Demandeurs ont également utilisé les réactions de transfert d'hydrogène en présence de cyclohexène et d'oxyde de palladium sur charbon. Selon le solvant réactionnel ou il n'y a pas de réaction ou alors la réduction est totale.

De même les réactions de transfert d'hydrogène avec le formiate d'ammonium et un catalyseur à base d'oxyde de palladium sur charbon conduisent soit à une hydrogénation totale (acétate d'éthyle, THF ou méthanol à chaud) soit à un mélange du produit désiré avec le produit de départ et avec des intermédiaires ainsi qu'avec des produits d'hydrogénation totale (méthanol ou méthanol/THF à température ambiante).

L'hydrogénolyse en présence de Nickel de Raney conduit à une hydrogénation totale. Au contraire, les réactions de déprotection en milieu acide fort (acide acétique/acide sulfurique) mènent à la décomposition du produit. Des conditions plus ménagées (HCl dilué ou H₂SO₄ dilué) ne permettent pas la déprotection des fonctions phénol.

La réaction en présence d'agents oxydants comme le DDQ ou le mélangeNaBrO₃/Na₂SO₄, conduit à des mélanges qui ne renferment pas de produit final.

Enfin, l'utilisation d'un acide de Lewis comme le chlorure d'aluminium donne des résultats variés : utilisé seul on observe la décomposition du mélange réactionnel, en mélange avec l'anisole, la réaction est très lente, et couplé avec la N, N-diméthylaniline selon la réaction décrite par T.Akiyama, H.Herofuji et S.Ozaki Tetrahedron Letters 32(10)(1991) 1321 - 1324, la débenzylation a été effectuée avec des résultats bien meilleurs.

En conséquence, la méthode de débenzylation la plus efficace selon l'invention, est celle qui utilise le couple halogénure d'aluminium /N, N- dialkylaniline et en particulier le couple chlorure d'aluminium /N, N- diméthylaniline.

Selon un mode d'exécution particulier de l'invention on prépare dans un premier temps un halogénure de 4-benzyloxybenzyle par réaction avec un halogénure de phosphore ou de soufre. Dans le cas précis du chlorure de 4-benzyloxybenzyle on effectue la chloration au moyen de chlorure de phosphore, d'oxychlorure de phosphore ou de chlorure de thionyle en présence de pyridine ou de triéthylamine ou de diméthylaniline.

La réaction de l'halogénure de 4-benzyloxy benzyle avec le phosphite de trialkyle s'effectue sans solvant. Le phosphite de trialkyle en excès sert de solvant réactionnel. La réaction est accélérée par la présence d'une petite quantité d'iodure de métal alcalin ou alcalino-terreux. De préférence, on fait réagir du chlorure de 4-benzyloxybenzyle et du phosphite de triéthyle en présence d'iodure de potassium.

On peut également dissoudre le phosphite de trialkyle dans un solvant polaire comme le diméthyl formamide, le diméthylacétamide ou le diméthylsulfoxyde et amener la formation de l'ylure par addition d'une base forte anhydre telle que le dimsyl sodium ou le terbutylate de potassium. La réaction nécessite un chauffage moins fort.

On peut en conséquence faire réagir simultanément l'halogénure de 4-benzyloxybenzyle, le phosphite de triakyle et l'iodure, ensuite préparer l'ylure et le faire réagir avec le 3,5-dibenzyloxybenzaldéhyde.

Selon l'invention, la réaction de l'hydroxybenzaldéhyde avec le phosphonate de 4-benzyloxybenzyle s'effectue soit directement en présence d'un réactif alcalin soit en préparant séparément l'ylure de phosphonate, en le faisant réagir avec l'hydroxybenzaldéhyde. La réaction est vigoureuse et requiert un temps de réaction plus court.

Dans la réaction directe on utilise un alcoolate de métal alcalin ou de métal alcalino-terreux comme un éthoxylate de lithium, de calcium ou de magnésium, un teramylate de potassium ou un terbutylate de potassium.

Le produit résultant de cette réaction est analysé par CCM et par HPLC pour déterminer s'il reste du phosphonate résiduel dans le produit d'aboutissement. On différencie ainsi le produit stilbénique, du composé aldéhydique et du phosphonate restant.

Pour finir, la réaction de déprotection et notamment la réaction de débenzylation s'effectue en milieu apolaire comme par exemple dans le dichlorométhane, le toluène, en utilisant le couple chlorure d'aluminium / N, N- diméthylaniline.

Les rendements globaux sont élevés et la pureté du Resvératrol permet d'obtenir une qualité pharmaceutique avec le minimum de réactions de purification. Une seule réaction de recristallisation avec acétone / heptane permet d'obtenir du (E) Resvératrol à plus de 98 % de pureté.

Le Resvératrol a été décrit comme possédant des propriétés thérapeutiques préventives ou curatives chez les humains et notamment chez la femme. On définit le paradoxe français comme résultant d'une nourriture méditerranéenne riche en graisses et en alcool, mais qui limite la survenue du cancer et des maladies cardiaques.

On a montré également que le Resvératrol avait des propriétés vasorelaxantes en inhibant la réponse contractile de l'aorte à la noradrénaline (C.K. Chem et Pace - Asiak/Gen. Pharmac (1996) 27,363-366) et était susceptible d'inhiber l'aggrégation des plaquettes et de limiter la coagulation (Goldberg, Clin. Chim Acta 237 (1995) 155).

Les exemples suivants illustrent l'invention. Ils ne la limitent en aucune façon.

### EXEMPLE I

Synthèse du 3,5-dihydroxyphényl-4-hydroxyphényl éthène (E) (ou Resvératrol) ou 3, 4',5-trihydroxy-*trans*-stilbène.

### Stade A

### Synthèse du phosphonate de 4-benzyloxy benzyle :

On introduit dans un ballon de 500 ml équipé d'un montage de distillation et d'une ampoule d'introduction 110 g (554,8 mol) de chlorure de 4-benzyloxybenzyle, 112 ml de phosphite de triéthyle et 1,1 g d'iodure de potassium.

On plonge le ballon de récupération dans un bain à 0° C pour récupérer les produits volatils et on chauffe le mélange réactionnel à 80° C pendant 30 minutes puis à 165° C pendant une heure. On suit la réaction en conduisant le chlorure d'éthyle formé en cours de la réaction dans le ballon de récupération.

Lorsque le dégagement de chlorure d'éthyle est terminé, on monte la température à 200° C pendant 15 minutes pour finir la réaction, tout en éliminant le phosphite de triéthyle en excès.

La masse obtenue est de 151g (452 mmol). Le rendement est de 81,4 %.
Le produit est suffisamment pur pour pouvoir être utilisé pour la réaction de Wittig-Horner subséquente.

Le 4-benzyloxybenzylphosphonate de diéthyle a été déjà décrit dans la littérature (Organic Réaction Vol 25 p 142). Le produit obtenu dans cette réaction est plutôt plus pur que celui précédemment décrit.

### Stade B

### Réaction de Wittig-Horner :

Dans un ballon de 500 ml, on introduit 45,1 g de 3,5-dibenzyloxybenzaldéhyde puis 47,7 g de 4-benzyloxybenzylphosphonate de diéthyle, dissous dans 200 ml de tétrahydrofuran.

On maintient sous agitation jusqu'à dissolution puis on ajoute 20,0 g de tert-butylate de potassium.

Le mélange est maintenu à température ambiante sous agitation pendant 3 heures. On évapore le mélange réactionnel à sec et le résidu solide obtenu est trituré dans 500 ml d'eau. On filtre ensuite sur verre fritté G2.

Le résidu recueilli est lavé à 2 reprises avec 100 ml d'eau à chaque fois.

On fait sécher le produit pendant une nuit à l'étuve sous vide à 50° C.
Masse obtenue 52,4 g (105 mmol), rendement 74,2 %.
L'évolution de la réaction est suivie en CCM (CH₂Cl₂/éther de pétrole 1 : 1 révélation UV/KMnO₄)
Tr du produit stilbénique 0,58
Tr de l'aldéhyde 0,22
Tr du phosphonate 0,00

On procède à une analyse et à une séparation par HPLC sur colonne MN Nucléosil C-18 de 100-5 µm.
Eluant CH3CN à 60 %, H2O 40 %
Rf Resvératrol 1,3 min.
Rf phosphonate 4,0 min.
Rf aldéhyde 12,0 min.

### Stade C

### Déprotection des groupes hydroxyle :

On dissout 100 g de 3,4',5-tribenzyloxy-Resvératrol dans 1000 ml de dichlorométhane ou de toluène sous agitation lente et en faisant barboter de l'azote dans la solution. On introduit d'abord la N,N-diméthylaniline (250 g) et ensuite par un ajout latéral le chlorure d'aluminium (210 g). On maintient le mélange réactionnel sous agitation à ~15°C. Après 60 minutes, on monte la température à 20-25°C pour compléter la débenzylation.
Après hydrolyse acide, le produit est extrait à l'acétate d'éthyle, le solvant est distillé en partie et le produit est insolubilisé par ajout de dichlorométhane, ensuite filtré et lavé au dichlorométhane.

Le produit ainsi obtenu est purifié par recristallisation dans acétone / heptane 1/2

Le Resvératrol cristallise de ce milieu sans retenir la moindre quantité de solvant. Le Resvératrol trans (E) ainsi obtenu fond à ~264°C en conformité avec la littérature. Son degré de pureté est déterminé par analyse HPLC. Il est supérieur à 98 %.

| Analyse centésimale : C₁₄H₁₂O₃ / PM = 228.25 | | | |
|---|---|---|---|
| | C % | H % | 0% |
| Théorique | 73,67 | 5,30 | 21,03 |
| Trouvé | 73,65 | 5,31 % | 21,09 |

SM (ES, m/e 229 M+1)
[1H]-RMN (CD₃OD-400 MHz) : 7.38 (d, J=8, 2H-C(3',5')), 6.98 et 6.83 (d, J=16, 2H vinyl), 6.79 (d,J=8, 2H-C(2',6')), 6.48 (d, J=2, 2H-C(2,6)) et 6.19 (t, J=2, 1 H-C(4)). [13C]-RMN (CD₃OD-100.6 MHz) : 160.55, 159.2, 142.2, 131.3, 130.3, 129.7, 127.9, 117.3, 106.6 et 103.5.

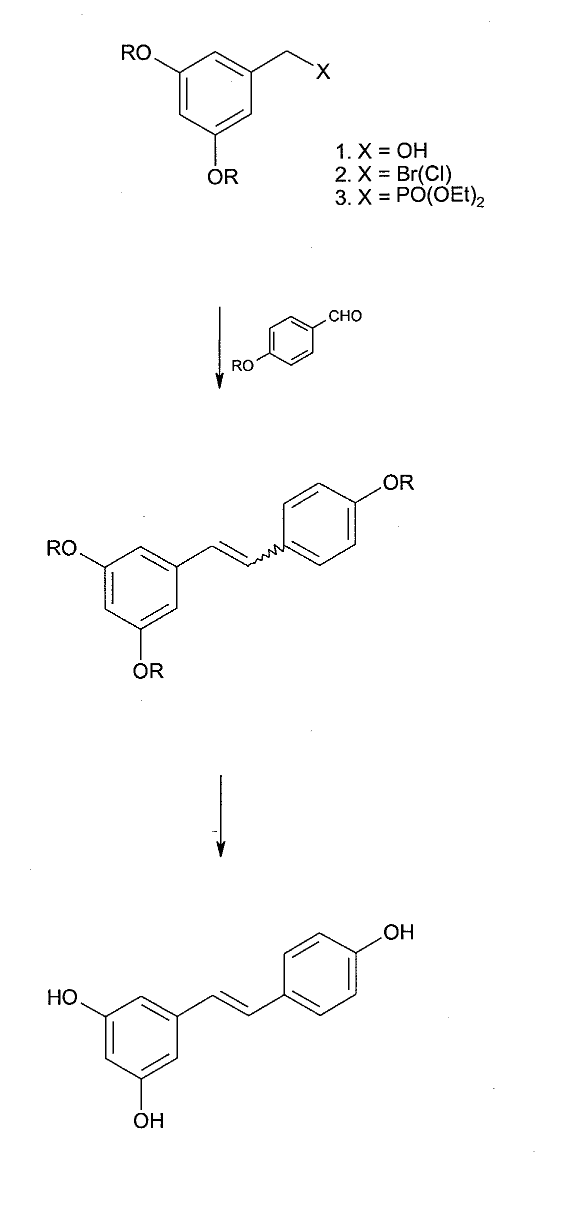

## Revendications

1. Nouveau procédé de synthèse du Resvératrol ou 3,5-dihydroxy-4'-hydroxy stilbène (isomère E) dans lequel on effectue la déprotection des groupes phénol au départ du 3,5-dibenzyloxy 4'-benzyloxy stilbène au moyen du couple halogénure d'aluminium / N, N dialkylaniline pour obtenir le 3,5-dihydroxy 4'-hydroxy stilbène avec un degré de pureté supérieure à 98 %.

2. Nouveau procédé de synthèse du Resvératrol selon la revendication 1, dans lequel l'halogénure d'aluminium est le chlorure d'aluminium.

3. Nouveau procédé de synthèse du Resvératrol selon la revendication 1, dans lequel la N,N-dialkylaniline est la diméthylaniline.

4. Nouveau procédé de synthèse du Resvératrol selon l'une des revendications 1 à 3, dans lequel les groupes phénols des synthons sont protégés par benzylation.

5. Nouveau procédé de synthèse du Resvératrol selon l'une des revendications 1 à 4, dans lequel la débenzylation est effectuée sur un composé aromatique à savoir le 3,5-dibenzyloxy-4'-benzyloxystilbène (isomère E).

6. Nouveau procédé de synthèse du Resvératrol dans lequel pour obtenir le 3,5-dibenzyloxy-4'-benzyloxystilbène (isomère E) on soumet un 4-benzyloxyphényl phosphonate de diéthyle à une réaction de Wittg-Horner sur le 3,5-dibenzyloxybenzaldéhyde.

7. Nouveau procédé de synthèse du Resvératrol selon l'une des revendications précédentes, dans lequel pour obtenir un 4-benzyloxyphénylphosphonate de dialkyle on fait réagir un halogénure de 4-benzyloxybenzyle avec un phosphite de trialkyle.

8. Procédé de synthèse du Resvératrol selon la revendication 7, dans lequel l'halogénure de benzyl oxybenzyle est un chlorure ou un bromure.

9. Procédé de synthèse du Resvératrol selon la revendication 7, dans lequel le phosphite de trialkyle est un phosphite de triéthyle.
